Europäisches Patentamt

European Patent Office

Office européen des brevets

(19)

(11) Publication number: **0 189 280**
**A2**

(12)

# EUROPEAN PATENT APPLICATION

(21) Application number: 86300289.5

(22) Date of filing: 17.01.86

(51) Int. Cl.⁴: **C 12 Q 1/68**

---

(30) Priority: **23.01.85 US 693936**

(43) Date of publication of application: **30.07.86 Bulletin 86/31**

(84) Designated Contracting States: **BE DE FR GB IT NL**

(71) Applicant: **Dekalb-Pfizer Genetics, 3100 Sycamore Road, Dekalb Illinois (US)**

(72) Inventor: **Cross, John William, 25-1 Brown's Lane, Old Lyme Connecticut (US)**

(74) Representative: **Moore, James William, Dr. et al, Pfizer Limited Ramsgate Road, Sandwich Kent CT13 9NJ (GB)**

---

(54) **Qualitative testing for nucleic acid.**

(57) An improved method for the detection of nucleic acid wherein the nucleic acid is detected in a crude tissue extract prepared directly on the test substrate by crushing tissue onto the substrate and subsequently carrying out the detection by standard means.

EP 0 189 280 A2

0189280

DPC 6819

## QUALITATIVE TESTING FOR NUCLEIC ACID

It is standard procedure to probe (with labeled nucleic acids) papers or other materials to which other nucleic acids under test have been bound (D. T. Denhardt, Biochem. Biophys. Res. Comm. 23, 641-646, 1966). The procedure usually involves the use of DNA bound to nitrocellulose in single stranded form, but procedures for attaching RNA to special papers (i.e. diazotized paper) have also been developed, and other proprietary sheet- and circle-type materials have become available. In these standard procedures the nucleic acid to be tested is normally purified before attaching it to the filter (M. Grunstein and D. S. Hogness, Proc. Nat. Acad. Sci. 72, 3961-3965, 1975).

A specialized procedure has also been developed for the hybridization of nucleic acids to the DNA of colonies of microorganisms. In this procedure, the colonies are pressed gently to the paper so that some of the cells attach to the dry paper. The cells are lysed, and the nucleic acid is released by treatment with alkali. The DNA released in this manner is then probed with labeled nucleic acid in a standard way. This procedure has not been applied to tissue juices of plants or animals, which also contain nucleic acids.

Another procedure has been developed by Dr. Ulrich Melcher (Plant Mol. Biol. 1, 63-73, 1981), in which nucleic acids contained in the cells of leaves have been cleared with solvents to remove interfering pigments and digested with a protease to remove proteins. This procedure allows for the probe to be applied directly to the treated leaves, and for the hybridization to occur in the leaves themselves. No attempt is made to remove the nucleic acids from the leaves before hybridization. This procedure suffers from the disadvantage that the treatment renders the leaves curled-up and brittle, and very difficult to handle. Moreover, it is very difficult to remove the pigments from the leaves quantitatively.

The present invention overcomes these shortcomings and for the first time provides a method for the rapid qualitative screening of plant and animal tissues for the presence of nucleic acids (DNA or RNA) homologous to tester (labeled probe) nucleic acids using the methods of nucleic acid hybridization.

An improved method for the detection of nucleic acid in a crude tissue extract comprising the steps of:

1)    contacting said extract with an adsorbent surface in order to bind said acid to said surface;

2)    denaturing said nucleic acid;

3)    washing said adsorbent surface to remove interfering substances;

4)    inactivating said adsorbent surface;

5)    hybridizing said bound nucleic acid by incubation with a specific, labeled, probe nucleic acid; and

6)    examining said bound nucleic acid for hybridization with said probe nucleic acid;

the improvement which comprises crushing tissue directly onto said adsorbent surface to form said crude tissue extract.

The method is applicable to both plant tissue and animal tissue. The method is also preferred wherein said nucleic acids to be detected are DNA or RNA, either of the tissues themselves or of viruses or bacteria or other micro-organisms or viroids infecting the tissues, and the method wherein said probe nucleic acid is labeled with radioactive atoms and said examining is carried out by radiographic means is preferred as is the method wherein said proble nucleic acid is labeled with nucleotides chemically modified to be recognizable by an enzyme and said examining is carried out by enzymatic means.

The method wherein said denaturing is carried out by contacting said nucleic acid with strong base especially NaOH or KOH is preferred, as is the method wherein said washing is carried out in buffered salt solutions followed by organic solvent and wherein said organic solvent is chloroform. The method is also preferred wherein after step 3), said adsorbent material is subjected to heat _in_ _vacuo_ prior to carrying out the remaining steps.

The method wherein said adsorbent surface is selected from cellulose, nitrocellulose, diazoben-zyloxymethyl cellulose, diazophenylthioether cellulose, diethylaminoethyl cellulose, epoxy-activated cellulose, triazinyl-activated cellulose, and nylon membranes is preferred. The method is also preferred wherein in step 4) said inactivation is by contacting active binding sites on said surface with a substance selected from non-specific denatured DNA, non-specific RNA, protein, ethanolamine, glycine, glycine methyl ester and glucosamine and especially wherein said DNA is selected from calf thymus DNA and _Escherichia_ _coli_ DNA.

A procedure has been devised for the rapid qualitative screening of tissues for the presence of nucleic acids (DNA or RNA) homologous to tester (labeled probe) nucleic acids using the methods of nucleic acid hybridization. The procedure consists of the following steps: 1. Crush or squeeze tissues or extract so that the juice is expressed on to a piece of nitrocellulose paper (or other sheet- or circle-type material often described as "membranes") which will bind the nucleic acids so that the paper absorbs the juice. 2. The paper is dried or fixed in ethanol or acid. 3. The paper is then treated with solvents to remove pigments and other interfering substances. 4. The paper is then treated with an alkaline solution to denature the DNA (This step may be omitted if the RNA of the tissue is to be probed). 5. Process the paper or membrane in the normal way for nucleic acid hybridizations using a labeled probe ([14C, 32P, 125I, biotinylated, etc.]-nucleic acid (DNA or RNA), prepared by in _vivo_ labeling, nick-translation, end-labeling, etc.). 6. Binding of the labeled-probe nucleic acid is then recorded by a standard, sensitive means, such as auto-radiography, fluoroscopy, enzymatic means, immunological means (as for biotinylated nucleic acid probe), etc. In addition to solid tissues which must be crushed as described, naturally liquid materials such as blood, pus, semen and endosperm as well as powders such as pollen can also be applied directly to the filter.

The nucleic acids expressed from the leaf or other tissue quickly soak into the matrix of the filter material, and dry or are fixed quickly, so that they are not degraded enough to prevent successful qualitative hybridization of probe nucleic acids. The procedure is rapid and reliable, so that many tissue samples can be screened for the presence of a particular nucleic acid sequence (which may be a gene, a DNA insertion element, a segment of repetitive DNA, a regulatory sequence, a virus, or any other segment of nucleic acid for which some suitable nucleic acid probe is available. The method will be useful in plant genetics research, in applied genetics (breeding), in human, animal and plant pathology (for diagnosis of human, animal and plant disease, or in breeding for resistance to disease), in forensic science for identification of species from fresh tissues or from extracts of dried leaves or dried tissue juices, and in genetic engineering experiments in which it is important to recognize the presence of certain pieces of DNA which are to be inserted into (or removed from) the cells of higher plants and animals using methods such as DNA vectors (Agrobacterium tumifaciens, or rhizogenes, Cauliflower mosaic virus or other DNA or RNA vectors), or other methods, such as cellular fusion, nucleic acid transformation, transfection, nucleic acid injection etc. It will be useful in the study of wide hybridizations, and in the recognition of the presence of recessive genes when their activity is not expressed due to the presence of dominant genes, etc. Unique tester genes can also be engineered into proprietary plant varieties and this method will then serve as a test of the identify of the variety.

The procedure of the present invention gives extremely satisfactory results if care is taken throughout to avoid cross contamination of non-hybridizing material by the more strongly-hybridizing material, thus producing a falsely positive hybridization signal. The procedure below has been found to fulfill this requirement.

1. Collection of material: As a matter of good procedure throughout the entire procedure material that is non-hybridizing or weakly-hybridizing should always be collected before strongly-hybridizing material. Any cutting instruments or gloves should be carefully cleaned or changed, and separate bags should be used for each sample type.

2. Crushing: During crushing, the sample is pressed with a hard instrument, such as a blunt probe or slug, against a nitrocellulose filter so that juice is released from the tissue, soaks into the nitrocellulose filter, and it is allowed to dry. Use of a separate filter disk for each sample will reduce the change of juice from one sample cross contaminating another. Alternatively, the tissue can be homogenized, with or without a suitable solvent, and applied to the filter. Gloves and instrument should be carefully cleaned or changed and new paper towel or other backing material for the nitrocellulose filter should be used.

3. Denaturation and fixation of the DNA: This is done by placing the nitrocellulose filters, sample-side up, on a double layer of Whatman 3MM or similar paper which has been saturated with solution, but with no excess solution beyond saturation, which can potentially flow over the surface of the 3MM paper and cross contaminate adjacent nitrocellulose filters. The precaution is necessary because some DNA moves through the filter and off the edges onto the underlying 3MM paper. Because of this movement of some DNA off the filters it is also desirable not to place adjacent filters too close to one another and to never place a filter on the 3MM paper on a position previously occupied by another filter. Forceps used to transfer filters between solutions should be cleaned. Typical solutions and treatment time for the samples are:

| Solution | Minutes |
|---|---|
| 0.5 M NaOH | 7 |
| 1 M Tris-Cl, pH 7.5 | 1 |
| 1 M Tris-Cl, pH 7.5 | 1 |
| 0.5 M Tris-Cl pH 7.5 + 1.5 M NaCl | 5 |

4. Chloroform wash: Wash each nitrocellulose filter on a Buchner funnel with two pieces of filter paper underneath. Allow the vacuum to remain on until all the chloroform has evaporated from the nitrocellulose filter. Clean the funnel and change the filter paper for the next sample.

5. NaCl rinse: Rinse each sample by submerging in a 0.3 M NaCl for a few moments and then air dry at 37°.

6. Baking: Bake in a vacuum oven at 78° for 3 3/4 hr.

7. Prehybridization: 0.1 x SCP (10 mM NaCl, 1.5 mM NaPi and 0.1 mM EDTA) pH 7.0 + 1 mg/100 ml of sheared calf thymus DNA heated to boiling for 10 min. to denature the DNA. The samples are then prehybridized in this solution at 65° for 30 min. in separate pouches to prevent cross contamination.

8. Hybridization: An appropriate amount of labeled probe is added to the hybridization solution below and heated to boiling for 10 min. to denature both the probe DNA and the calf thymus DNA. The samples are then hybridized 12.5 hr at 65° in separate pouches for each sample type.

| ml | Solution |
|----|----------|
| 5 | 10 x Denhardt's solution* |
| 7.5 | 20 x SSC |
| 10 | 50% dextran sulfate |
| 0.2 | 25% SDS |
| 24 | $H_2O$ |
| 2.5 | boiled sheared calf thymus DNA |
| appropriate amount | boiled probe |

9.  Rinse and washes:  Remove samples from separate hybridization pouches and rinse in separate containers in 1 SSC + 0.5% SDS.  Then wash 4 x for 1/2 hr in the same solution at 65°.  Air dry.

10. Place against X-ray film with enhancement screen and expose for appropriate time at -70°.


## EXAMPLE 1

Two 13.7 cm diameter disks of pure nitrocellulose (S&S type BA85) were ruled with a 5 x 5 grid of lines with a soft pencil.  To the squares of the filters were applied crudely expressed leaf juices of three species of plant, Zea mays, Glycine max and Sorghum bicolor, as follows:

Fresh leaves were brought from the greenhouse and cut into conveniently sized pieces, about 10 cm in length.  A coin was used to smash the leaf tissue onto the central area of each square so that tissue juice was deposited thereon (a fresh segment of leaf was used for each square).  The smashing of the tissue was accomplished by rubbing the leaf back and forth with the edge of the coin, and pressing hard against the clean, hard surface against which the nitrocellulose paper rested.  The resulting expressed juice ran onto

* Biochem. Biophys. Res. Comm. 23, 641-646, 1966.

the filter and covered the center of the square, staining it green and brown. Care was taken to ensure that the juice from one square did not run onto other ruled squares. The juice then soaked into the paper and was allowed to air-dry at 37 C.. The leaf juice was applied to the squares with one species of leaf per row and at least four squares from each species per disk. The filters were then cut in halves along one of the ruled lines perpendicular to the rows of each species, so that each half-filter contained at least two spots from each species.

The filter-halves were placed in sequence on stacks of two blotter papers saturated with the following solutions: NaOH (0.5 N, 7 min.), Tris.HCl (1.0 M, pH 7.4, 1 min.), Tris.HCl (1.0 M, pH 7.4, 1 min.), NaCl (1.5 M) + Tris.HCl (0.5 M, pH 7.4 5min), and air-dried, all at room temperature. The blotters contained enough of each of these solutions to saturate the filter-halves with solution, but not enough to cause the DNA to be washed away before it was fixed to the filter. The filter-halves were then extracted with chloroform until almost all of the green pigment was removed, and air-dried again. The filter halves were next baked in a vacuum over at 78°C for 3 hr 45 min. and prehybridized for 30 min. at 65°C in 0.1x SCP (1x SCP = 100 mM NaCl, 15 mM sodium phosphate, 1mM EDTA, pH 7.0) plus 1 mg of sheared-, unlabeled-, pure non-specific DNA (salmon testis DNA for the E. coli probe and E. coli DNA for the Z. mays and S. bicolor probes), which blocked non-specific reactive groups on the filter surfaces. The filters were then ready for hybridization.

Pure samples of DNA from <u>E. coli</u>, <u>Z. mays</u> and <u>S. bicolor</u> were then nick translated using alpha-[32-P]-deoxy-ATP as the label. These 32-P-labeled DNA samples were then used as hybridization probes to test the efficacy of the squish hybridization technique. To accomplish this, three of the filter halves were separately sealed into one of three plastic bags, along with on of the above described DNA probes. The basic hybridization solution contained 5 mL of 10x Denhardt's solution, 7.5 mL of 20x SSC, 10 mL of 50% (w/v) dextran sulfate 0.2 mL of 25 % (w/v) SDS, 24 mL of sterile water and 2.5 mL of 2 mg/mL boiled, sheared carrier DNA (of a type corresponding to that used in the prehybridization step described immediately above). To this solution was added the specific probe DNA (1.5 million DPM per bag). Hybridization was carried out overnight at 65°C in 5 mL of the above solution per bag. The filters were then removed from the bags and rinsed once in 1x SSC plus 0.1% SDS, and then washed four more times in the same solution at 65°C (for 30 min. each change) with gentle shaking. The filters were then air dried.

The filters were taped to a paper backing and the hybridization was visualized by radioautography with Kodak X-OMAT film (overnight exposure at -70°C with a DuPont Cronex "Extra Life, Lighting Plus" intensification screen). The film was developed and photographed. A positive result was scored when a spot was darkened on the film which corresponded in position and shape with the position and shape of the spot produced by the tissue juices expressed onto the nitrocellulose filter.

The results are indicated in table below.

TABLE I

| Source of Leaf Material or DNA | Reaction Results (Source of Probe DNA) | | |
|---|---|---|---|
| | E. coli | Z. mays | S. bicolor |
| 1.   G. max | - | - | - |
| 2.   Z. mays | - | + | - |
| 3.   S. bicolor | - | + | - |

## EXAMPLE 2

Leaf materials of the species listed in Table II were collected from the field surrounding the Central Research Plant Growth Facility in Groton, CT and tissue juices were expressed onto nitrocellulose filters as described in the previous example.  Thus juice each species or tissue type was expressed onto a specific spot on the filter which subsequently could be scored qualitatively for hybridization.  The treatment of the filters and the hybridization was also as described in the example above. [32-P]-labeled probe DNA was prepared using standard nick translation techniques using purified plasmid pSH1, which had been isolated by standard cloning techniques from the genomic DNA of S. bicolor using the vector pBR322. pSH1 contains 2.7 kb of sorghum DNA ligated into the HindIII site of pBR322.  The procedures for obtaining this clone of DNA are familiar to one skilled in the art of DNA cloning.

Positive hybridization to the probe DNA of the various spots on the filter was scored only when dark spots appeared on the X-ray film in corresponding positions, as described in the example above. The results are given in Table II.

TABLE II

| Species | Variety | Tissue | Reaction to pSHL Probe | Replications |
|---|---|---|---|---|
| Sorghum bicolor | N3494 | leaf | + | 12 |
| | | embryo | + | 4 |
| | White Khafir | leaf | + | 2 |
| | Hol62 | leaf | + | 3 |
| Zea mays | A188 | leaf | – | 3 |
| | 7277 | leaf | – | 3 |
| Zea diploperennis | | leaf | – | 3 |

The experiment indicates that the technique detects DNA from all varieties of Sorghum bicolor tested but none of Zea. Therefore the method can be species-specific when the appropriate labeled probe DNA is applied.

EXAMPLE 3

In this case nylon filters are prepared as were the nitrocellulose filters in Example 1. Fresh samples of leaf from turnip plants are obtained. Some of these plants are known to be infected with cauliflower mosaic virus, some are known to be free of infection, and others are suspected of being infected. These samples are squeezed against ruled squares on the filters as in Example 1 to express juice. The filters are dried and then processed as in that example except that the prehybridization step is modified by the addition of 10% bovine serum albumin to the solution and by extending the prehybridization to 12 hr. A sample of purified cauliflower mosaiac virus DNA is nick-translated as in Example 1 and, following the procedure detailed in that example, this nick-translated viral DNA is used to probe the filters for hybridization. The filters are washed and exposed to X-ray films as described in the first example.

This procedure will then produce dark spots on the X-ray film where the spot deposited on the filter by the tissue juice does contain cauliflower mosaic virus, since juice from the leaves of plants known to be infected will produce dark spots on the film following this procedure, while the areas of the film corresponding to plants which were not infected will not become darkened.

### EXAMPLE 4

In this case fresh samples of leaf from tabacco plants are obtained. Some of these plants are known to be infected with tobacco mosaic virus, some are known to be free of infection, and others are suspected of being infected. These samples are squeezed against ruled squares on the filters as in Example 1 to express juice. The filters are dried and then processed as in that example through the step of prehybridization with non-specific (E. coli) DNA, but omitting the step of denaturation with NaOH. A sample of purified tabacco mosaic virus RNA is labeled with 32-P by polynucleotide kinase, a procedure known to those skilled in the art. This labeled viral RNA is then used to probe the filters for hybridization by the procedure detailed in Example 1. The filters are washed and exposed to X-ray films as described in the first example.

This procedure will then produce dark spots on the X-ray film where the spot deposited on the filter by the tissue juice does contain tobacco mosaic virus. It will then be known which plants were infected by tobacco mosaic virus, since juice from the leaves of plants known to be infected will produce dark spots on the film following this procedure, while the areas of the film corresponding to plants which were not infected will not become darkened.

## EXAMPLE 5

In this case nitrocellulose filters are prepared as in Example 1. Samples of leaf and other plant tissues of unknown species suspected of containing Cannabis sativa are also applied to the filters after they are rehydrated in a minimal quantity of 0.5 N NaOH. Also appied to the filters are samples from authentic leaf tissue of Cannabis sativa, which will serve as positive controls. These samples are squeezed against ruled squares on the filters as in Example 1 to express the absorbed 0.5 N NaOH (which will dissolve the DNA in the tissue). The filters are dried and then processed as in that example through the step of prehybridization with non-specific (E. coli) DNA. A sample procedure as was the species-specific clone of DNA (to be cloned from Cannabis by the same procedure as was the species-specific clone of DNA employed in Example 2 is labeled with 32-P by nick translation. This labeled DNA is then used to probe the filters for hybridization by the procedure detailed in Example 1. The filters are washed and exposed to X-ray films as described in the first example.

This procedure will then produce dark spots on the X-ray film where the spot deposited on the filter by the tissue extract does contain DNA from authentic <u>Cannabis sativa</u>. It will be known which plants are definitely <u>Cannabis sativa</u>, since extracts from the leaves of plants known to be <u>Cannabis sativa</u> will produce dark spots on the film following this procedure, while the areas of the film corresponding to other species of plant will not become darkened.

## EXAMPLE 6

In this case nitrocellulose filters are prepared as in Example 1. Samples of beef liver are obtained from the slaughter house. Other samples of liver are obtained from markets. These samples are pressed against ruled squares on the filters using a laboratory press so that the tissue juices are expressed. Care is taken to ensure that the spots from the tissue juices do not run into other ruled squares of the filters. The filters are dried and then processed as in Example 1 through the step of prehybridization with non-specific (E. coli) DNA. A sample of a purified clone of species-specific DNA (to be cloned from bovine DNA by the same procedure as was the species-specific clone of DNA employed in Example 2 is labeled with 32-P by nick translation following the procedure detailed in Example 1. This nick-translated DNA is used to probe the filters for hybridization. The filters are washed and exposed to X-ray films as described in the first example.

This procedure will then produce dark spots on the X-ray film where the spot deposited on the filter by the tissue juice does contain bovine DNA. It will then be known which livers were authentic beef liver, since only juice from authentic beef livers will produce dark spots on the film following this procedure, while the area of the film corresponding to livers of other species will not become darkened.


### EXAMPLE 7

In this case a sample of pure DNA Staphylococcus aureus is prepared and a series of clones are made from restriction fragments of this DNA using procedures known to those skilled in the art. These clones are examined for their species-specificity. A clone is identified which is highly species specific for Staphylococcus aureus and which displays no cross-hybridization to human DNA.

Nitrocellulose filters are prepared as in Example 1. Samples of pus are obtained from patients suspected of infection with Staphylococcus aureus. Other samples of pus are obtained from patients which are known to have been infected with Staphylococcus aureus. These samples are allowed to dry on ruled squares of the filters. Care is taken to ensure that the spots from the pus do not run into other ruled squares of the filters. The filters are dried and then processed as in Example 1 through the step of prehybridization with non-specific (calf thymus) DNA. A sample the DNA of this clone is purified and nick translated with biotin-conjugated nucleotides to produce a DNA probe which is labeled with biotin. This nick-translated DNA is used to probe the filters for hybridization. The filters are washed and exposed to appropriate antibodies capable of recognition of the biotin-labeled nuclcotides contained in the DNA probe, as supplied and recommended by the kit manufacturer or the biotin-conjugated nucleotides. The antibodies binding to the filter are then visualized by enzymatic test reactions which are commercially available.

This procedure will then produce pigmented areas on the filter where the pus deposited on the filter does contain Staphylococcus aureus organisms. It will then be known which patients were infected with Staphylococcus aureus bacillus, since only pus from patients infected with Staphylococcus aureus will cause pigmented areas to form on the membrane filter following this procedure.

## EXAMPLE 8

In this case nitrocellulose filters are prepared as were the filters in Example 1. Fresh samples of peach leaves from are obtained.. Some of these plants are known to be infected with the mycoplasma which causes Peach X-Disease, some are known to be free of infection, and others are suspected of being infected. These leaf samples are squeezed against ruled squares on the filters as in Example 1 to express juice. The filters are dried and then processed as in that example. A sample of authentic DNA is prepared from the Peach X-disease and is nick-translated as in Example 1. Following the procedure detailed in that example, this nick-translated mycoplasma DNA is used to probe the filters for hybridization. The filters are washed and exposed to X-ray films as described in the Example 1.

This procedure will then produce dark spots on the X-ray film where the spot deposited on the filter by the tissue juice does contain the mycoplasma of Peach X-Disease. It will then be known which plants were infected with this disease, since juice from the leaves of plants known to be infected will produce dark spots on the film following this procedure, while the areas of the film corresponding to plants which were not infected will not become darkened.

PC 6819

## CLAIMS

1.  An improved method for the detection of nucleic acid in a crude tissue extract comprising the steps of:

1)  contacting said extract with an adsorbent surface in order to bind said acid to said surface;

2)  denaturing said nucleic acid;

3)  washing said adsorbent surface to remove interfering substances;

4)  inactivating said adsorbent surface;

5)  hybridizing said bound nucleic acid by incubation with a specific, labeled, probe nucleic acid; and

6)  examining said bound nucleic acid for hybridization with said probe nucleic acid;

characterised in that tissue is crushed directly onto said adsorbent surface to form said crude tissue extract.

2.  The method of claim 1 wherein said tissue is plant tissue.

3.  The method of claim 1 wherein said tissue is animal tissue.

4.  The method of claim 1 wherein said nucleic acid to be detected is DNA.

5.  The method of claim 1 wherein said nucleic acid to be detected is RNA.

6.  The method of claim 1 wherein said probe nucleic acid is labeled with radioactive atoms and said examining is carried out by radiographic means.

7.    The method of claim 1 wherein said probe nucleic acid is labeled with nucleotides chemically modified to be recognizable by an enzyme or antibody and said examining is carried out by enzymatic means.

8.    The method of claim 1 wherein after step 3), said adsorbent material is subjected to heat in vacuo prior to carrying out the remaining steps.

9.    The method of claim 1 wherein after step 3), said adsorbent material is subjected to heat in an inert atmosphere prior to carrying out the remaining steps.

10.    The method of claim 1 wherein in step 4) said inactivation is by contacting active binding sites on said surface with a substance selected from non-specific denatured DNA, non-specific RNA, protein, ethanolamine, glycine, glycine methyl ester and glucosamine.